# EUROPEAN PATENT SPECIFICATION

(11) **EP 2 945 666 B1**
(45) Date of publication and mention of the grant of the patent: **09.10.2019**
(21) Application number: 14701927.7
(22) Date of filing: 14.01.2014
(51) Int. Cl.: A61M 11/00, A61F 9/00, B05B 11/02, G01F 11/02, A61M 5/20, A61M 5/24, A61M 5/30, A61M 5/315, A61M 11/06, A61M 5/31, A61M 15/00, A61M 5/14

(54) **MEDICAMENT DELIVERY DEVICE**
MEDIKAMENTENABGABEVORRICHTUNG
DISPOSITIF D'ADMINISTRATION DE MÉDICAMENT

(30) Priority: 15.01.2013 SE 1350046; 15.01.2013 US 201361752600 P
(43) Date of publication of application: 25.11.2015
(73) Proprietor: SHL Medical AG, 6302 Zug (CH)
(72) Inventor: EGERSTRÖM, Johan, 132 41 Saltsjö-Boo (SE); SÖDERLUND, Marcus, 115 40 Stockholm (SE); RONQUIST, Nils, 114 22 Stockholm (SE)
(86) International application number: PCT/EP2014/050569
(87) International publication number: WO 2014/111370

(56) References cited:
- WO-A1-2006/126902
- US-A1- 2008 177 246
- US-A1- 2009 118 669
- US-A1- 2010 114 038
- US-A1- 2012 136 306
- US-A1- 2012 296 276

## Description

### TECHNICAL AREA

The present invention relates to a medicament delivery device and in particular a device capable of delivering a number of predetermined metered doses.

### BACKGROUND OF INVENTION

There is a large number of devices for delivering medication for absorption in the respiratory system or for medicament delivery to the eyes and one strong trend is to develop devices for self-administration. Traditionally oral or nasal medication was intended for treatment of diseases in the trachea, bronchi and/or lungs, and in particular acute or chronic symptoms of asthma. However, much research is now performed on administration of medicament via the lungs in treatment of many other ailments due to beneficial absorption rate and reduced side effects. Further, oral administration provides a greater safety than delivery devices using needles because of the problem with possible contamination from a used needle. A similar situation exists for delivery of medicament to the eyes of a patient.

However, there is a number of medicament compositions that are not compatible with existing devices and there are further a number of drawbacks with existing devices. For example aerosol dispensers utilise a propellant which provides the force to aerosolize the medicament in liquid form. However, this requires a special type of pressurised canister, usually of metal, which increases the cost for manufacturing and affects the environment.

One example of a device which could be used for metered oral administration is described in the document WO 2011/043712. The disclosed device comprises a plunger rod, which, when driven by a pre-tensioned helical spring, forces a stopper into a cartridge containing medicament, whereby a dose of medicament is delivered from an administration member. The administration member could be a mouth piece or a nasal piece.

With the device according to WO 2011/043712, it is possible to set a dose by turning a spring force tensioning member at a distal end of the device. This action also tensions a spiral spring for delivering a dose. The setting and delivery of doses may be made a number of times until the medicament container is empty.

A drawback with the device of WO 2011/043712 is that there is no indication that a dose has been set and that the device is ready for dose delivery. This may be a drawback where the device user either is not very used to handling the device so that he/she is uncertain that the device has been handled properly for making it ready for dose delivery. Also for experienced users, it is advantageous to be provided with a positive indication that the handling steps prior to dose delivery have been performed correctly.

There is thus still a need for devices where a number of doses may be delivered and where handling steps need to be performed prior to each dose delivery to be provided with information and/or verification that the handling steps have been performed such that the device is ready for dose delivery.

### BRIEF DESCRIPTION OF INVENTION

As used herein, the term "liquid" encompasses all solutions, suspensions, emulsions, oils, gels and so forth, which generally behave as liquids at operating temperatures. The term explicitly includes solid compositions dissolved or dispersed in a liquid carrier. Materials behaving as highly viscous liquids are also included.

In the present application, when the term "distal part/end" is used, this refers to the part/end of the medical delivery device, or the parts/ends of the members thereof, which during use of the device is located the furthest away from the delivery site of the patient. Correspondingly, when the term "proximal part/end" is used, this refers to the part/end of the device, or the parts/ends of the members thereof, which under use of the device is located closest to the delivery site of the patient.

The aim of the present invention is to remedy the drawbacks of the state of the art devices. This is obtained by a medicament delivery device according to the features of the independent patent claim 1. Preferable embodiments of the invention form the subject of the dependent patent claims.

The present invention pertains to a medicament delivery device for delivering metered doses of medicament. It comprises at least one generally elongated tubular chassis, the chassis having opposite distal and proximal ends.

Inside said chassis, a threaded plunger rod is arranged. The plunger rod is arranged to cooperate with a drive nut, which is threadedly connected to the threaded plunger rod.

The device is further provided with a drive member. This drive member is rotatably connected to a spring force tensioning member. In a preferred embodiment, the spring force tensioning member is accessible outside the distal end of the chassis. It is however to be understood that the spring force tensioning member may be placed on other locations on the chassis.

Further, the drive member is connected to the drive nut via an drive member extension such that said drive member extension and said drive member are operably interconnected for providing a rotational lock of the drive member extension in an opposite direction when said drive member is rotated by said spring force tensioning member.

The drive member extension and said drive nut are operably interconnected for providing a rotational lock of the drive nut in relation to the drive member extension.

The device is further arranged with spring force member, that in one preferred embodiment is in the form of a spiral spring. It is however to be understood that other types of spring force member may be used for the same function.

The spring force member has a first end connected to the drive member and a second end connected to a fixed point on the chassis such that said spring force member is tensioned when said spring force tensioning member and said drive member are rotated.

In order to handle the plunger rod, a guide nut is arranged with guide ledges which may cooperate with longitudinal grooves of the plunger rod for providing a rotational lock but allowing a longitudinal movement of the plunger rod in relation to the guide nut.

In order to facilitate the handling of the medicament delivery device, a manually operated activation means is releasably interconnected to said drive member extension by engagement means for providing a rotational locking of the drive member extension in any direction when said drive member is rotated and said spring force member is tensioned by handling said spring force tensioning member for setting the device ready for delivery of a dose of medicament.

According to the present invention, an indicator mechanism is operably connected to said drive member and arranged to visibly indicate to a user of the device when said spring force tensioning member has been operated such that the device is ready for dose delivery.

In this manner, the user is informed by the indicator mechanism when the spring force tensioning member has been operated to an extent that the device is ready for use. The user is thus provided with positive information and can be assured that when the indication is visible, the device is ready for dose delivery.

According to one solution, the indicator mechanism may comprise a surface operably connected to said drive member, which surface is arranged with a noticeable indication member, the indicator mechanism further comprising an opening in said chassis for viewing said surface. Thus, when the drive member is turned, the surface is positioned in line with the opening, and is thus visible to the user.

According to a preferable solution of the invention, the surface may be operably arranged at said drive member such that said indication member is visible in said opening when said spring force tensioning member has been operated, i.e. showing that the device is ready for dose delivery. In this manner, the indicator becomes visible only when the spring force tensioning member, and thus the drive member, has been turned such that a dose has been set and the device is ready for dose delivery.

In order to be clearly distinctive and easily visible to a user, the indication member comprises a distinct colour. This colour may for example be a clear green colour, indicating a ready device in a positive way. However, the colour may be chosen such that it is a clear alert, such as orange, yellow, possibly also fluorescent.

In addition to a colour, or instead of a colour, the indication member may comprise indicia. The indicia could for example be a check mark, letters, words like "OK", a graphic symbol or the like, just to mention a few.

In order to elevate the visibility further, the opening may be arranged with a magnifying lens or the like, where the indication member is even more clearly visible.

According to a further aspect of the invention the medicament delivery device is an inhalation device, an eye spray device or an injector.

These and other aspects of, and advantages with, the present invention will become apparent from the following detailed description of the invention and from the accompanying drawings.

### BRIEF DESCRIPTION OF DRAWINGS

In the following detailed description of the invention, reference will be made to the accompanying drawings, of which
- Fig. 1: is a perspective view of a medicament delivery device comprising the present invention,
- Fig. 2: is an exploded view of the proximal part of Fig. 1,
- Fig. 3: is a cross-sectional view of the proximal part of Fig. 2,
- Fig. 4: is a detailed view of a distal area of the proximal part of Fig. 2,
- Fig. 5: is a perspective view of a distal area of the proximal part of Fig. 2,
- Fig. 6: is a perspective view of a distal part of the device of Fig. 1,
- Fig. 7: is an exploded view of the distal part of Fig. 6,
- Fig. 8: is a cross-sectional view of the distal part of Fig. 6,
- Figs. 9-16: are detailed views of components comprised in the distal part of Fig. 6,
- Figs. 17-18: are detailed views showing different functional states.

### DETAILED DESCRIPTION OF THE INVENTION

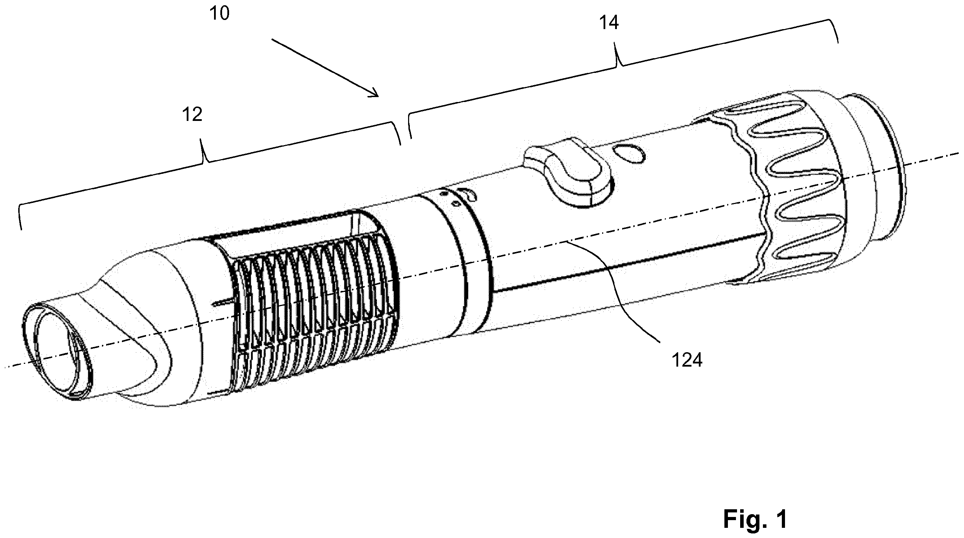

One embodiment of a device shown in the drawings comprises a generally elongated body 10. The body 10 is in the embodiment shown divided into two parts; a proximal part 12 and a distal part 14, Fig. 1.

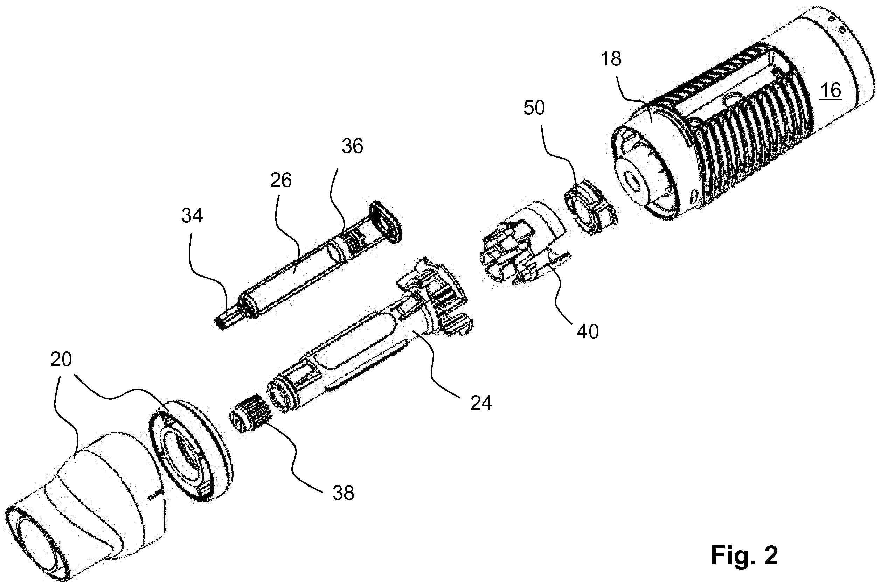

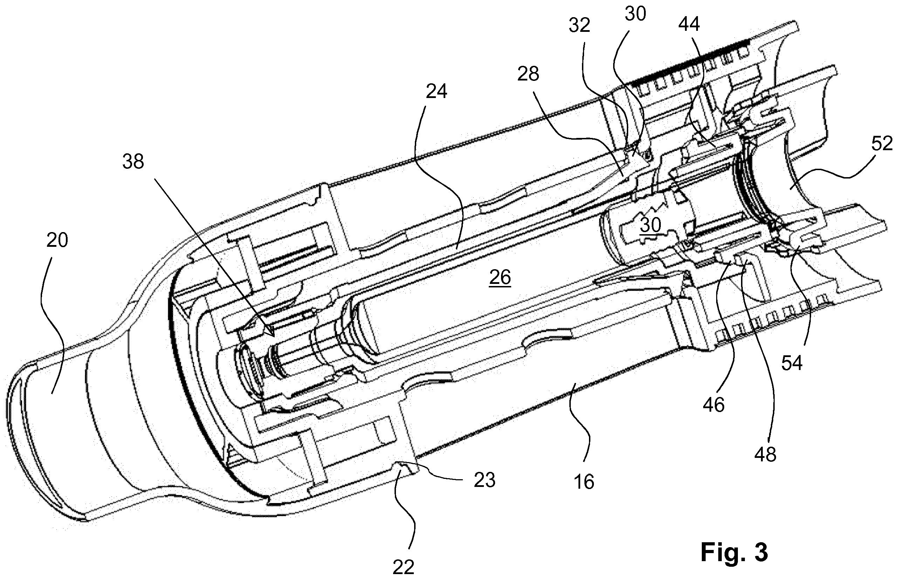

The proximal part 12 comprises generally tubular housing part 16, Fig. 2. A proximal end of the housing part 16 is arranged with an attachment area 18, Fig. 2, onto which a medicament delivery member 20 can be releasably attached. The attachment means between the medicament delivery member 20 and the housing 16 could for example be ledges 22 that snap into corresponding grooves 23, Fig. 3. It is however to be understood that other types of attachment means could be utilized. The medicament delivery member 20 is in the embodiment shown a mouthpiece through which a user inhales when a dose of medicament is to be delivered. It is however to be understood that other types of medicament delivery members could be used, such as nasal pieces, eye pieces and even injection needles and the like.

The proximal part 12 is further arranged with a generally elongated tubular medicament container holder 24, Fig. 2, arranged to accommodate a medicament container 26. The medicament container holder 24 is arranged with generally radially flexing legs 28, Fig. 3, with outwardly extending ledges 30 that fit into recesses 32 in the housing part 16.

The medicament container 26 is in the embodiment shown arranged with a proximal neck 34 and a movable stopper 36 inside the container. At the proximal end of the medicament container holder 24, a nebulizing nozzle 38 is arranged, Figs. 2 and 3. The nebulizing nozzle 38 contains a chip (not shown) with a plurality of micro channels capable of creating an aerosol of droplets of medicament.

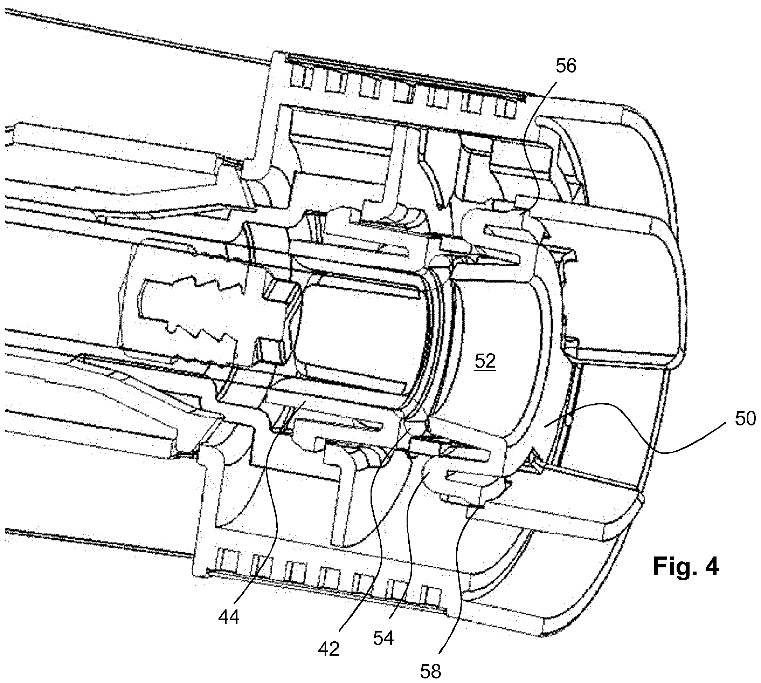

A medicament container guide member 40, Figs. 2 and 3, is arranged with a central passage and is intended to be pushed onto the medicament container 26 from its proximal end and into contact with an annular ledge 42 (Fig. 4) at a distal end of the medicament container 26, Fig. 2. The medicament container guide member 40 is arranged with a number of proximally extending arms 44, Fig. 3, arranged generally radially flexible and having outwardly extending protrusions 46. These protrusions 46 are arranged to grip around an annular ledge 48 of the housing for locking the medicament container guide member 40 and thus the medicament container 26 in the radial direction.

A medicament holder locking member 50, Fig. 4, is arranged to be pushed into the distal end of the medicament container holder 24, and moved in contact with a distally directed end surface of the medicament container 24. The medicament holder locking member 50 is arranged with a central passage 52 and is provided with a number of generally radially flexible tongues 54 having outwardly extending protrusions 56, Fig. 4. These protrusions 56 are arranged to fit into recesses 58 on inner surfaces of the medicament container guide member 40 such that when the medicament holder locking member 50 is in place, the medicament container 26 is locked in the axial direction.

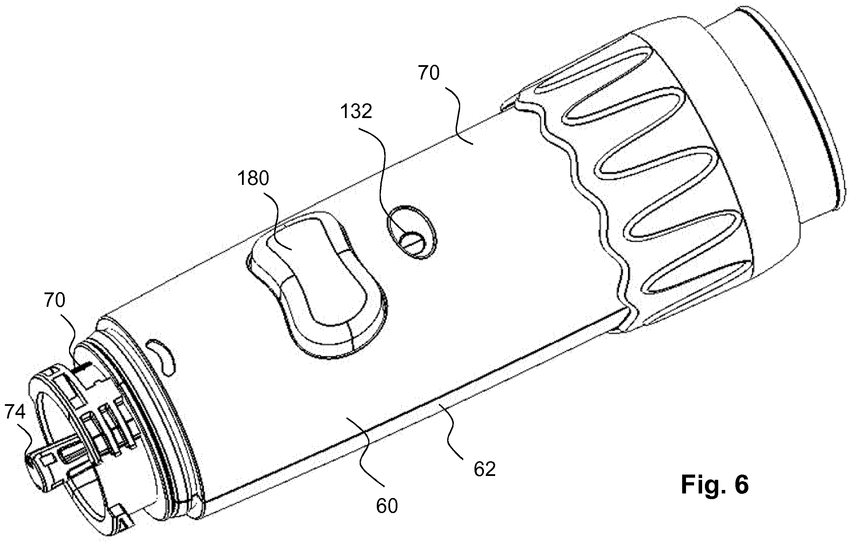

The distal part 14 is arranged with a housing. In the embodiment shown the housing is designed as two housing halves 60, 62, Fig. 6. It is however to be understood that the housing may be arranged in other ways and in more parts without departing from the scope of the invention.

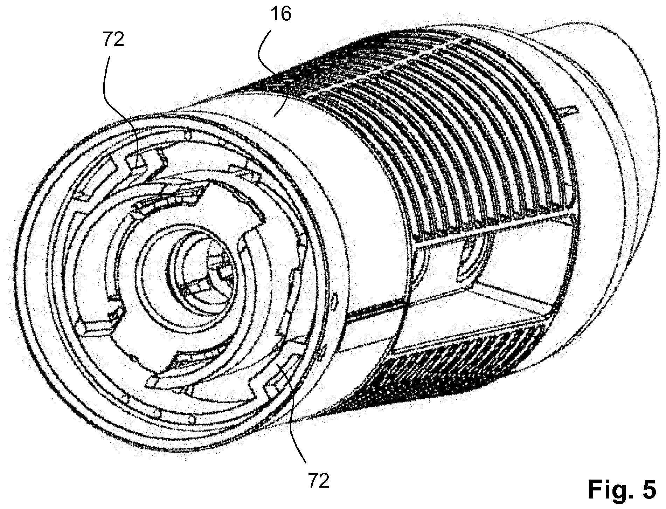

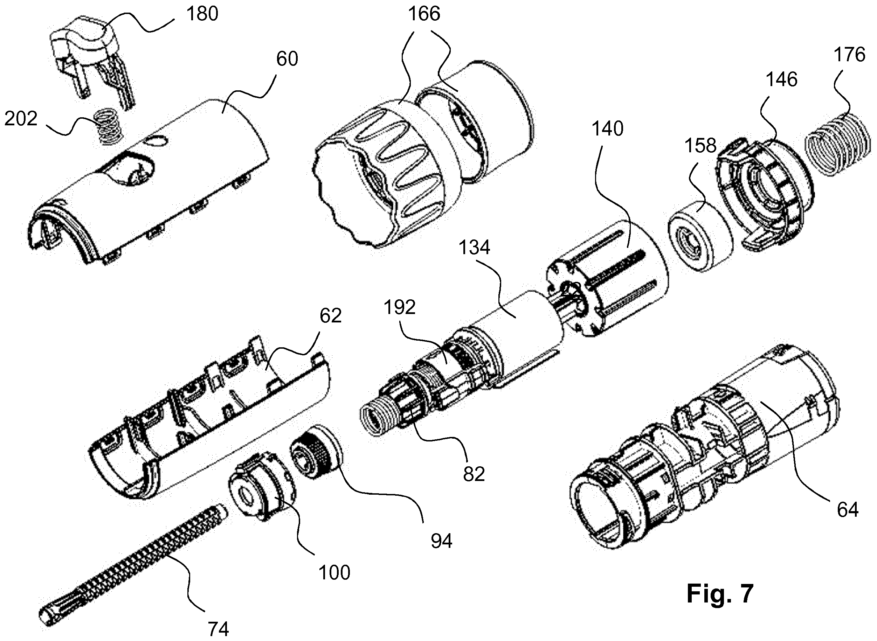

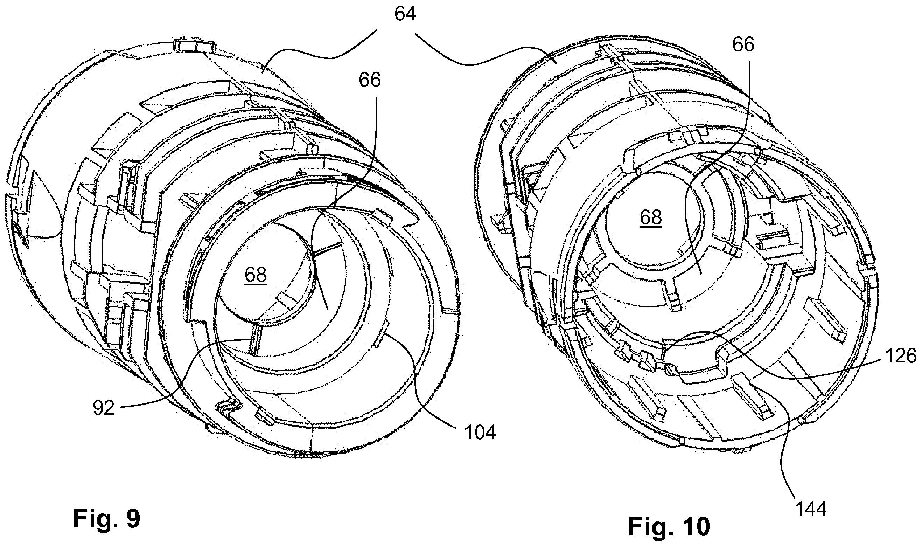

Inside the housing parts, a generally tubular chassis 64 is arranged, Figs. 7, 9, 10. It is arranged with a transversal interior wall 66, Figs. 9 and 10. The interior wall 66 is further provided with a central passage 68. At a proximal end of the chassis, attachment means 70, Fig. 6, are arranged, in the embodiment shown as grooves for a bayonet connection. In this context it is to be understood that other types of attachment means may be employed, such as threads, snap-on fittings or the like, all within the imagination of the person skilled in the art. The attachment means 70 of the chassis 64 are intended to interact with corresponding attachment means 72 on the distal end of the generally tubular housing part 16, Fig. 5.

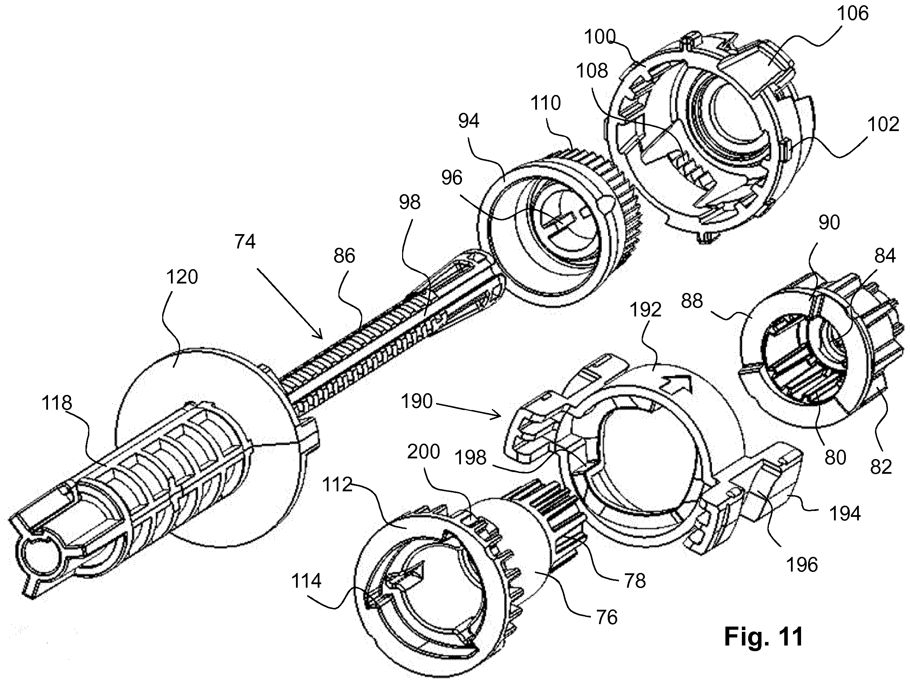

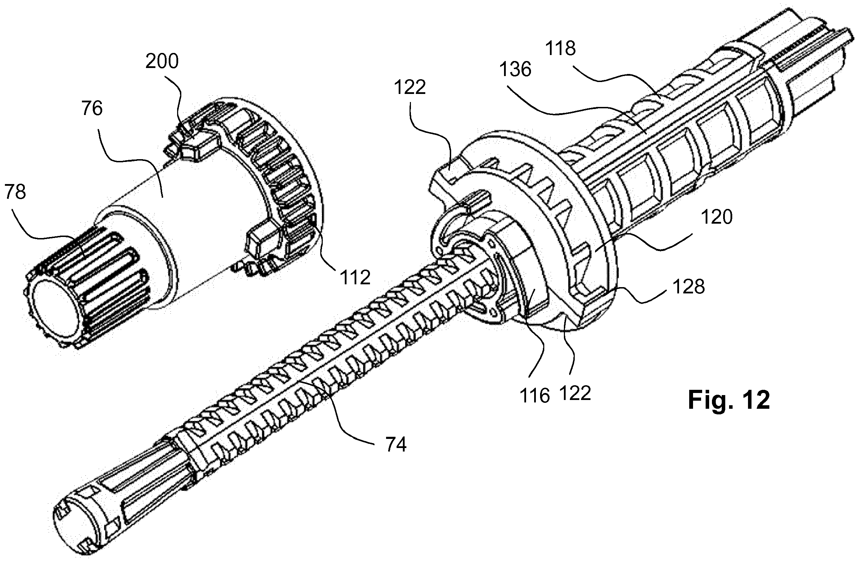

Coaxially arranged inside the chassis 64 and extending in the longitudinal direction of the device is an elongated plunger rod 74, Figs. 6 and 7. The plunger rod 74 is intended to be in contact with a stopper arranged in the medicament container. The plunger rod 74 is arranged inside a so called drive member extension 76, Figs. 11 and 12, having a generally tubular shape. A proximal end of the drive member extension 76 is arranged with spline grooves 78 on its outer surface, Figs. 11 and 12. The spline grooves 78 mate with corresponding spline ridges 80 on an inner surface of a generally tubularly shaped drive nut 82, Fig. 11.

The inner surface of the drive nut 82, Fig. 11, is further provided with threads 84, which cooperate with threads 86 on the outer surface of the plunger rod 74. The drive nut 82 is further arranged with an annular ledge 88 with a distally directed end surface having a number of slanting wedge-shaped surfaces 90. The proximally directed surface of the interior wall 66 of the chassis 64 is arranged with corresponding slanting wedge-shaped surfaces 92, Fig. 9.

The plunger rod 74 is further arranged through a guide nut 94, Fig. 11. The guide nut 94 is arranged with guide ledges 96 which cooperate with longitudinal grooves 98 of the plunger rod 74, Fig. 6, providing a rotational lock but allowing a longitudinal movement of the plunger rod 74 in relation to the guide nut 94. The guide nut 94 fits into a guide nut lock member 100, which is attached to the chassis 64 via radially outwardly extending protrusions 102 fitting into recesses 104 in the interior surface of the chassis 64, Fig. 9. The nut lock member 100 is arranged with tongues 106 that are arranged flexible in the generally radial direction. The inner surfaces of the flexible tongues 106 are arranged with wedge-shaped teeth 108, Fig. 11, extending in the radial direction. The teeth 108 of the tongues 106 are intended to engage corresponding wedge-shaped teeth 110 arranged around the circumference of the guide nut 94.

Further, the drive member extension 76 is arranged with a ring-shaped part 112, Fig. 11, with a hollow interior, where the inner surface of the interior is arranged with transversal stop ledges 114. These stop ledges 114 cooperate with flexible arms 116 arranged at a proximal end of a generally elongated drive member 118, Fig. 12.

However the stop ledges 114 and flexible arms 116 are arranged to provide a unidirectional lock such that the drive member 118 may only be rotated in one direction in relation to the drive member extension, where the flexible arms 116 slide over the stop ledges 114. In the other direction, the ends of the flexible arms 116 abut the stop ledges 114, thereby blocking their relative rotation. The drive member 118 is further arranged with a disk-shaped member 120, Fig. 12. On the proximally directed side of the disk-shaped member 120, two stop ledges 122 are arranged diametrically on opposite sides of a longitudinal axis 124, Fig. 1, of the device. The stop ledges 122 are arranged to co-act with corresponding stop ledges 126, Fig. 10, arranged in the interior of the chassis 64 for limiting the rotation of the drive member 118.

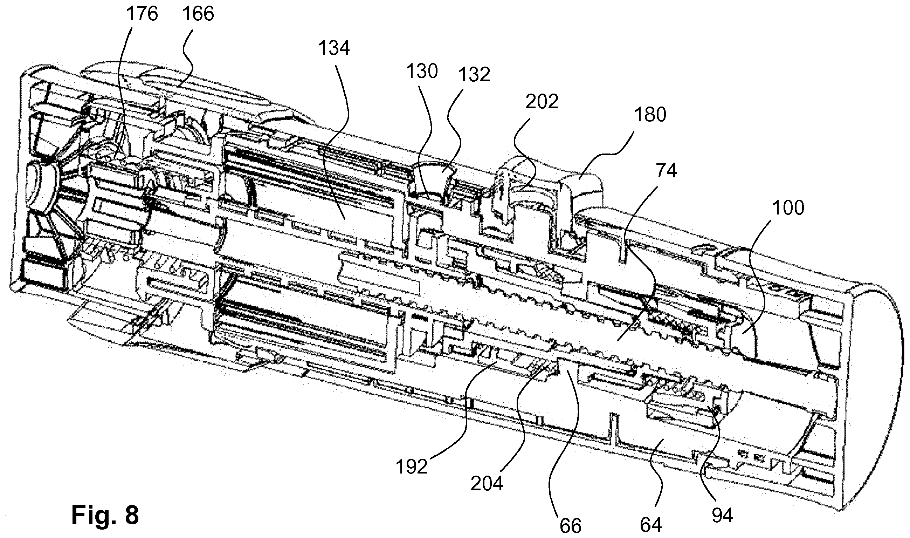

Further, according to the invention, the drive member 118 is arranged with a generally radially outwardly directed surface 128, Fig. 12, preferably arranged with a certain noticeable colour or indicia to be used as an indicator of the state of the device, as will be described. The indicator surface 128 could be arranged with a bright colour, such as bright green or bright orange, or could have certain signs or letters like a check mark or "OK", just to mention a few feasible indicators. In connection with this surface 128, the chassis is arranged with an opening or a transparent area 130, Fig. 8, through which the indicator surface may be visible. Also the housing part 60 is arranged with an opening or a transparent area 132, Fig. 6, where the indicator surface 128 may be viewed. The opening 132 may further be arranged with a magnifying lens so as to increase the visibility of the indicator.

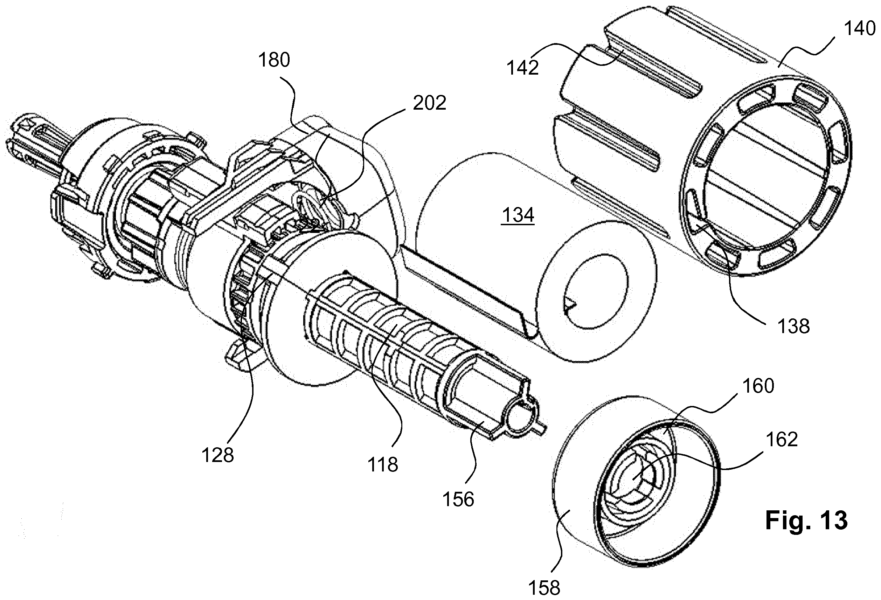

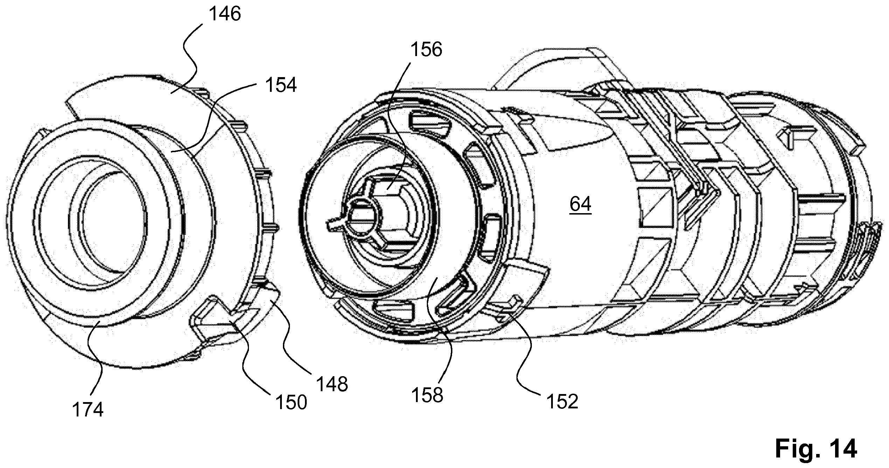

A spiral drive spring 134, Fig. 13, is arranged around the drive member 118 and attached with an inner end in an elongated slit 136, Fig. 12, in the drive member 118 and with the other end in a slit 138 in a spring house 140, Fig. 13. The spring house 140 is in turn placed inside the distal part of the chassis 64, Fig. 10, and held fixed in relation to the chassis 64 by longitudinal grooves 142 in the outer surface of the spring house 140 fitting with corresponding ridges 144. The spring house 140 is held in place in the chassis 64 by a spring house cover 146, Fig. 14, which is attached to the chassis 64 by proximally directed arms 148 with passages 150, which accommodate ledges 152 on the outer surface of the chassis 64. The spring house cover 146 is further arranged with a distally directed tubular member 154.

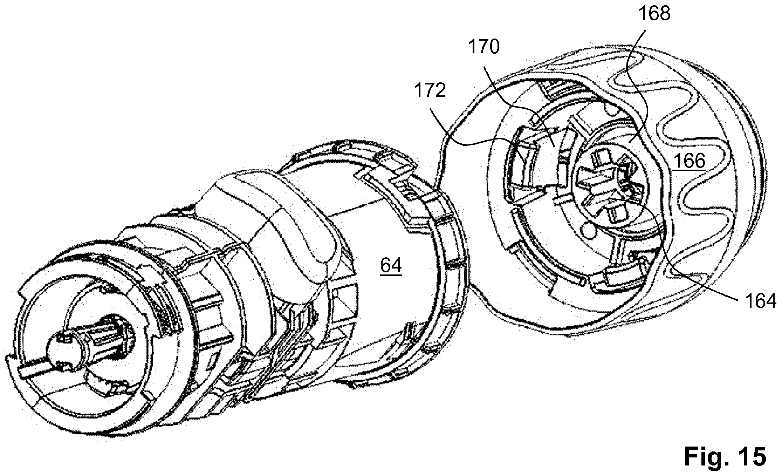

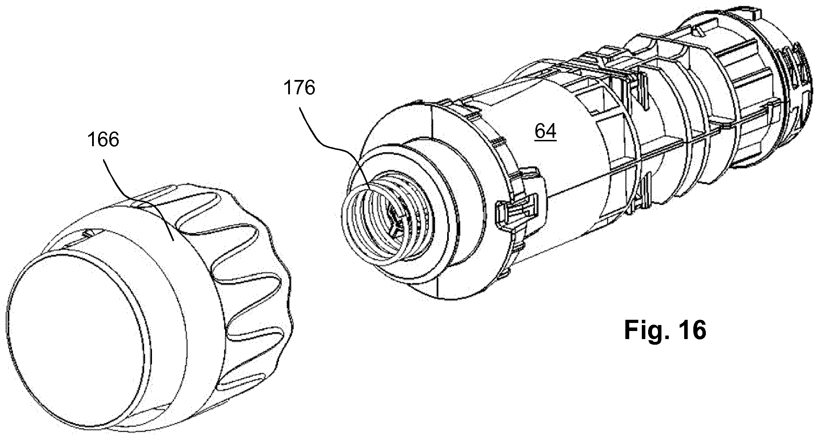

The distal end of the drive member 118 extends into the tubular member 154 of the spring house cover 146. The distal end of the drive member 118 is arranged with a number of longitudinally extending ledges 156, Fig. 13. A generally tubular guide member 158, Fig. 13, 14, is further provided, having an end wall 160. The end wall 160 is arranged with a central passage 162 with a shape corresponding to a cross-section of the distal end of the drive member 118 with the ledges 156, forming a keying function. The distal end of the drive member 118 with the ledges 156 are further arranged to fit into a corresponding recess 164 on a spring force tensioning member 166, Fig. 15, such that the spring force tensioning member is operably connected, e.g. rotationally connected, to the drive member. The recess 164 is positioned on a central post 168 inside the spring force tensioning member 166, Fig. 15. The spring force tensioning member 166 is further arranged with a number of proximally directed arms 170, which arms 170 are flexible in the generally radial direction. The free ends of the arms 170 are arranged with inwardly extending ledges 172, which ledges 172 are designed to fit around an annular ledge 174, Fig. 14, arranged on the circumference of the tubular member 154. A spring 176, Fig. 16, is further arranged between the end wall 160 of the guide member 158 and an interior, proximally directed wall of the spring force tensioning member 166, urging the latter in the distal direction.

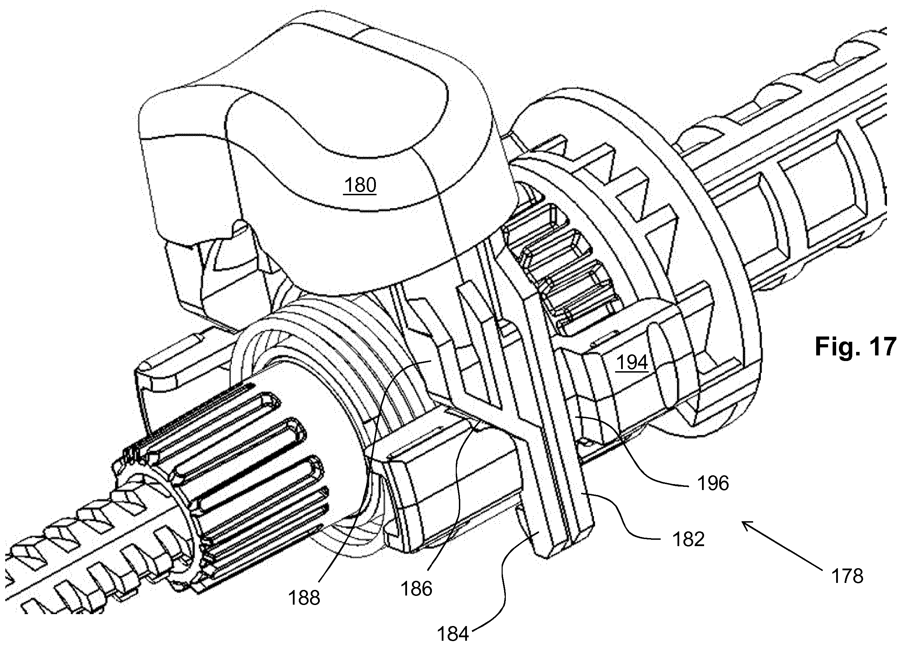

Further, an activation mechanism 178, Fig 17, is arranged in the device. It comprises an activation button 180 extending through an opening of one of the housing parts 60. The activation button 180 comprises two inwardly extending arms, 182, one on each side of the longitudinal direction 124 of the device. Each arm 182 is arranged with a first surface 184 facing in the proximal direction. The first surface 184 transitions into a second surface 186, inclined with respect to the first surface 184. The second surface 186 thereafter transitions into a third surface 188 generally parallel with the first surface 184.

The arms 182 are in contact with a dose activator 190, Fig, 11, comprising a ring-shaped body 192, Fig. 11, surrounding a part of the drive member extension 76. The ring-shaped body 192 is arranged with two elongated posts 194, extending in the longitudinal direction of the device. Each post is arranged with a groove 196, in which grooves the arms 182 of the activation button 180 fit, Fig. 17.

A distally directed end surface of the ring-shaped body 192 is arranged with a number of circumferentially directed stop ledges 198, Fig. 11, the function of which will be described below. The stop ledges 198 are to interact with stop ledges 200 on an outer surface of the drive member extension 76. A compression spring 202, Fig. 13, is arranged between an interior surface of the activation button 180 and an outer side surface of the chassis 64 for urging the activation button 180 towards an extended position. Further a second compression spring 204, Fig. 8, is arranged between a proximally directed end surface of the ring-shaped body 192 of the dose activator 190 and a distally directed surface of the transversal wall 66 for urging the ring-shaped body 192 in the distal direction and in engagement between the stop ledges 198 of the ring-shaped body 192 and the stop ledges 200 of the drive member extension 76.

The device is intended to function as follows. An appropriate medicament container 26 is placed in the medicament container holder 24 together with the medicament container guide member 40 such that its arms snap in engagement with the medicament holder. The medicament holder is then inserted into the proximal housing part 16. Then the medicament holder locking member 50 is pushed in engagement with the medicament container guide member 40. The proximal housing part 12 is then connected to the distal housing part 14 and the chassis 64 via the attachment means 70, 72. The distal end of the medicament container guide member 40 is designed such that it engages the outer surface of the nut lock member 100, whereby the tongues 106 are pressed radially inwards such that the teeth 108 of the tongues 106 engage the teeth 110 of the guide nut 94, whereby the guide nut 94 becomes rotationally locked.

When a dose is to be set and delivered the spring force tensioning member 166 is operated, e.g. rotated. In order to connect the spring force tensioning member 166 to the dose setting mechanism, the spring force tensioning member 166 is pushed in the proximal direction against the force of the spring 176. The spring force tensioning member 166 and the drive member 118 are then connected in that the ledges 156 fit into the recess 164. Thus, when the spring force tensioning member 166 is rotated, the drive member 118 is also rotated. The rotation of the drive member 118 causes the spiral drive spring 134 to be tensioned from an initial state where it was pre-tensioned during manufacture of the device.

During rotation, the flexible arms 116 of the drive member 118 move out of contact with the stop ledges 114 of the ring-shaped member 112 of the drive member extension 76 until they are moved in contact with subsequent stop ledges 114. The drive member 118 is prevented from being rotated back because the contact of the flexible arms 116 with the stop ledges 114.

Further, the drive member extension 76 is in turn prevented from rotating because the stop ledges 198 of the dose activator 192 are in contact with the stop ledges 200 on the drive member extension 76.

The spring force tensioning member 166 is rotated until the stop ledge 122 of the drive member 118 comes in contact with the corresponding stop ledge 126 of the chassis 64. This ensures that the user cannot turn the spring force tensioning member 166 beyond a pre-set position. Therefore a too large dose cannot be set. Now that a dose is set the device is ready for use. A positive feed-back to the user that the device is ready is shown in the opening or window 132 of the device because the turning of the drive member has positioned the indication surface 128 in line with the opening or window 132 of the device. The user is then able to notice the colour or symbol that is an indication that the device is ready for use.

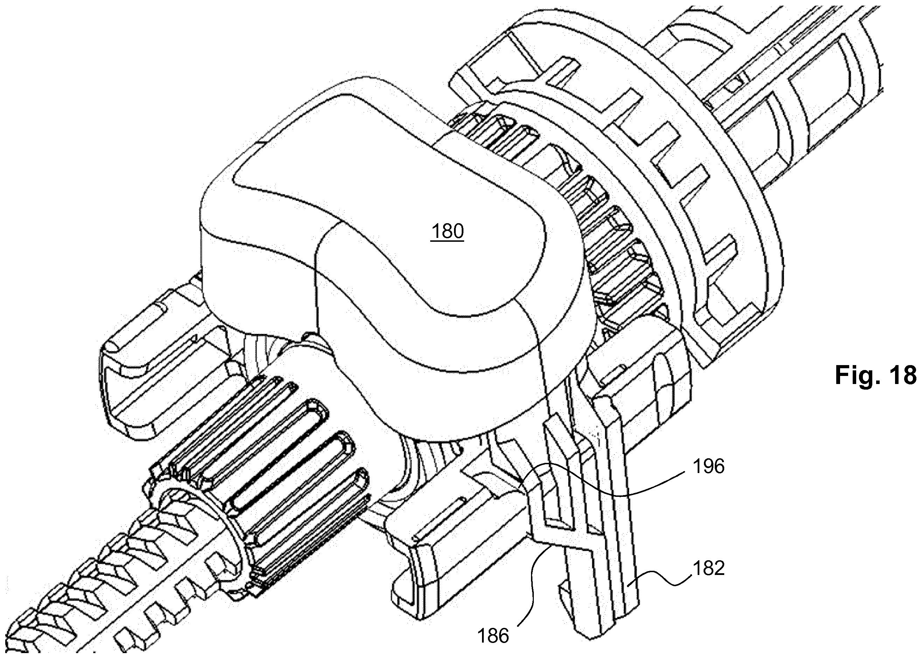

The user may now position the medicament delivery device at the delivery site and may manually activate the medicament delivery device by pressing the activation button 180 into the device against the force of the return spring 202. The movement of the activation button 180 causes the arms 182 to slide in the grooves 196 of the posts 194. After a certain movement, the inclined second surface 186 is moved in contact with a distally directed surface of the groove 196 and thereafter the third surface 188, Fig. 18. This contact of the second and third surfaces forces the ring-shaped body 192 in the proximal direction. This in turn causes the stop ledges 198 of the dose activator 190 to move out of contact with the stop ledges 200 of the drive member extension 76. The drive member extension 76 and thereby the drive member 118, because of the connection between the flexible arms 116 and the stop ledges 114, are now free to rotate by the force of the spring 134, and due to the splines connection between the drive member extension 76 and the drive nut 82, the latter is also rotated.

Due to the rotation of the drive nut 82, which is in threaded engagement with the threads 86 of the plunger rod 74, and because of the rotational lock of the plunger rod with the guide nut 94, the plunger rod 74 is axially advanced, which causes it to move the stopper 36 inside the medicament container 26 and to force the medicament through the medicament delivery member, e.g. the nebulizing nozzle 38 and the mouth-piece 20.

It is to be understood that the embodiment described above and shown in the drawings is to be regarded only as a non-limiting example of the invention and that it may be modified in many ways within the scope of the patent claims.

## Claims

1. A metered droplet medicament delivery device comprising:
- a generally elongated tubular chassis (64) having opposite distal and proximal ends;
- a drive mechanism arranged within the chassis, comprising
∘ a threaded plunger rod (74);
∘ a drive nut (82) threadedly connected to the threaded plunger rod;
∘ a drive member (118) rotatably connected to a spring force tensioning member (166) accessible outside the distal end of the chassis (64), said drive member (118) being connected to the drive nut (82) via a drive member extension (76), wherein said drive member extension (76) and said drive member (118) are operably interconnected to provide a unidirectional rotational lock of the drive member (118) relative to the drive member extension (76) when said drive member (118) is rotated by said spring force tensioning member (166), wherein said drive member extension (76) and said drive nut (82) are operably interconnected for providing a rotational lock of the drive nut (82) in relation to the drive member extension (76), the distal end of the drive member (118) is arranged with a number of longitudinally extending ledges (156), a generally tubular guide member (158) is further provided, having an end wall (160), the distal end of the drive member (118) with the ledges (156) is further arranged to fit into a corresponding recess (164) on the spring force tensioning member (166), such that the spring force tensioning member (166) is rotationally connected to the drive member (118), a spring (176) is further arranged between the end wall (160) of the guide member (158) and an interior, proximally directed wall of the spring force tensioning member (166), urging the latter in the distal direction;
∘ a guide nut (94) arranged with guide ledges (96) which cooperate with longitudinal grooves (98) of the plunger rod (74) for providing a rotational lock but allowing a longitudinal movement of the plunger rod in relation to the guide nut,;
- the spring force tensioning member (166) accessible outside the distal end of the chassis (64) and operably connected to the drive mechanism;
- a spring force member (134) having a first end connected to the spring force tensioning member through the drive mechanism and a second end connected to a fixed point on the chassis (64) such that said spring force member (134) is tensioned when said spring force tensioning member (166) is operated;
- a manually operated activation means (178, 180, 182, 192) releasably interconnected to said drive mechanism and configured to interact with said drive mechanism such that after the spring force member (134) is tensioned, operation of said spring force tensioning member (166) is prevented; wherein the device further comprises an indicator mechanism (128, 132) operably connected to said drive mechanism and arranged to visibly indicate to a user of the device when said spring force tensioning member (166) has been operated such that the device is ready for dose delivery, and wherein the drive member 118 is arranged with a generally radially outwardly directed surface 128 arranged with indicia to be used as an indicator of a state of the device.

2. A metered droplet medicament delivery device according to claim 1, wherein said manually operated activation means (178, 180, 182, 192) is releasably interconnected to said drive member extension by engagement means (198, 200) for providing a rotational lock of the drive member extension (76) in any direction when said drive member (118) is rotated and said spring force member (134) is tensioned by operation of said spring force tensioning member (166) for setting the device ready for delivery of a dose of medicament.

3. A metered droplet medicament delivery device according to claim 1, wherein the first end of said spring force member (134) is connected to the drive member (118).

4. A metered droplet medicament delivery device according to claim 1, wherein said indicator mechanism (128, 132) is operably connected to said drive member (118) and arranged to visibly indicate through the chassis to the user of the device when said spring force tensioning member (166) has been operated such that the device is ready for dose delivery.

5. A metered droplet medicament delivery device according to claim 4, wherein said indicator mechanism (128, 132) comprises a surface (128) operably connected to said drive member (118), which surface (128) is arranged with a noticeable indication member, and wherein the chassis is arranged with an opening.

6. A metered droplet medicament delivery device according to claim 5, wherein said surface (128) is operably arranged to said drive member (118) such that said indication member is visible in said opening when said spring force tensioning member (166) has been operated such that the device is ready for dose delivery.

7. A metered droplet medicament delivery device according to claims 4 or 6, wherein said indication member comprises a distinct colour.

8. A metered droplet medicament delivery device according to claims 4 or 6, wherein said indication member comprises a symbol.

9. A metered droplet medicament delivery device according to claim 5, wherein said opening is arranged with a magnifying lens.

10. A metered droplet medicament delivery device according to claim 1, wherein said device is an inhaler.

11. A metered droplet medicament delivery device according to claim 1, wherein said device is an eye spray device.

12. A metered droplet medicament delivery device according to claim 1, wherein said device is an injector.

## Patentansprüche

1. Medikamentenabgabevorrichtung für dosierte Tröpfchen, umfassend:
- ein im Wesentlichen längliches, röhrenförmiges Gehäuse (64), das gegenüberliegend ein distales und proximales Ende aufweist;
- einen Antriebsmechanismus, der innerhalb des Gehäuses angeordnet ist, umfassend:
- eine Kolbenstange (74) mit Gewinde;
- eine Antriebsmutter (82), die über das Gewinde mit der Kolbenstange mit Gewinde verbunden ist;
- ein Antriebselement (118), das drehbar mit einem Federkraftspannungselement (166) verbunden ist, das außerhalb des distalen Endes des Gehäuses (64) zugänglich ist, wobei das Antriebselement (118) mittels einer Antriebselementverlängerung (76) mit der Antriebsmutter (82) verbunden ist, wobei die Antriebselementverlängerung (76) und das Antriebselement (118) miteinander wirkverbunden sind, um eine einseitig gerichtete Verdrehsicherung des Antriebselements (118) in Bezug auf die Antriebselementverlängerung (76) bereitzustellen, wenn das Antriebselement (118) durch das Federkraftspannungselement (166) gedreht wird, wobei die Antriebselementverlängerung (76) und die Antriebsmutter (82) miteinander wirkverbunden sind, um eine Verdrehsicherung der Antriebsmutter (82) in Bezug auf die Antriebselementverlängerung (76) bereitzustellen, das distale Ende des Antriebselements (118) mit einer Anzahl von sich längsseitig erstreckenden Absätzen (156) versehen ist, ferner ein im Wesentlichen röhrenförmiges Führungselement (158) vorgesehen ist, das eine Stirnwand (160) aufweist, ferner das distale Ende des Antriebselements (118) mit den Absätzen (156) so angeordnet ist, dass es in eine entsprechende Aussparung (164) an dem Federkraftspannungselement (166) passt, sodass das Federkraftspannungselement (166) mit dem Antriebselement (118) drehverbunden ist, ferner eine Feder (176) zwischen der Stirnwand (160) des Führungselements (158) und einer inneren, proximal ausgerichteten Wand des Federkraftspannungselements (166) angeordnet ist, die letzteres in die distale Richtung drängt;
- eine Führungsmutter (94), die mit Führungsabsätzen (96) versehen ist, die mit Längsnuten (98) der Kolbenstange (74) zusammenwirken, um eine Verdrehsicherung bereitzustellen, aber eine Längsbewegung der Kolbenstange in Bezug auf die Führungsmutter zu ermöglichen;
- das Federkraftspannungselement (166), das außerhalb des distalen Endes des Gehäuses (64) zugänglich und mit dem Antriebsmechanismus wirkverbunden ist;
- ein Federkraftelement (134), das ein erstes Ende, das mit dem Federkraftspannungselement durch den Antriebsmechanismus verbunden ist, und ein zweites Ende aufweist, das mit einem festen Punkt auf dem Gehäuse (64) verbunden ist, sodass das Federkraftelement (134) gespannt wird, wenn das Federkraftspannungselement (166) betätigt wird;
- ein von Hand betätigtes Aktivierungsmittel (178, 180, 182, 192), das lösbar mit dem Antriebsmechanismus verbunden und ausgestaltet ist, mit dem Antriebsmechanismus derart in Wechselwirkung zu treten, dass, nachdem das Federkraftelement (134) gespannt wurde, die Betätigung des Federkraftspannungselements (166) verhindert wird;
wobei die Vorrichtung ferner einen Anzeigemechanismus (128, 132) umfasst, der mit dem Antriebsmechanismus wirkverbunden und angeordnet ist, einem Benutzer der Vorrichtung optisch anzuzeigen, wenn das Federkraftspannungselement (166) betätigt wurde, sodass die Vorrichtung für die Abgabe einer Dosis bereit ist, und wobei das Antriebselement (118) mit einer im Wesentlichen radial nach außen gerichteten Fläche (128) versehen ist, die mit Kennzeichen versehen ist, die als Anzeige für einen Zustand der Vorrichtung zu verwenden sind.

2. Medikamentenabgabevorrichtung für dosierte Tröpfchen nach Anspruch 1, wobei das von Hand betätigte Aktivierungsmittel (178, 180, 182, 192) mittels eines Eingriffsmittels (198, 200) zum Bereitstellen einer Verdrehsicherung der Antriebselementverlängerung (76) in jeder Richtung lösbar mit der Antriebselementverlängerung verbunden ist, wenn das Antriebselement (118) gedreht wird und das Federkraftelement (134) durch Betätigung des Federkraftspannungselements (166) gespannt wird, um die Vorrichtung zur Abgabe einer Medikamentendosis einzustellen.

3. Medikamentenabgabevorrichtung für dosierte Tröpfchen nach Anspruch 1, wobei das erste Ende des Federkraftelements (134) mit dem Antriebselement (118) verbunden ist.

4. Medikamentenabgabevorrichtung für dosierte Tröpfchen nach Anspruch 1, wobei der Anzeigemechanismus (128, 132) mit dem Antriebselement (118) wirkverbunden und angeordnet ist, um dem Benutzer der Vorrichtung optisch durch das Gehäuse anzuzeigen, wenn das Federkraftspannungselement (166) betätigt worden ist, sodass die Vorrichtung für die Abgabe einer Dosis bereit ist.

5. Medikamentenabgabevorrichtung für dosierte Tröpfchen nach Anspruch 4, wobei der Anzeigemechanismus (128, 132) eine Fläche (128) umfasst, die mit dem Antriebselement (118) wirkverbunden ist, wobei die Fläche (128) mit einem wahrnehmbaren Anzeigeelement versehen ist, und wobei das Gehäuse mit einer Öffnung versehen ist.

6. Medikamentenabgabevorrichtung für dosierte Tröpfchen nach Anspruch 5, wobei die Fläche (128) funktionell an dem Antriebselement (118) angeordnet ist, sodass das Anzeigeelement in der Öffnung sichtbar ist, wenn das Federkraftspannungselement (166) betätigt worden ist, sodass die Vorrichtung für die Abgabe einer Dosis bereit ist.

7. Medikamentenabgabevorrichtung für dosierte Tröpfchen nach Anspruch 4 oder 6, wobei das Anzeigeelement eine deutlich erkennbare Farbe umfasst.

8. Medikamentenabgabevorrichtung für dosierte Tröpfchen nach Anspruch 4 oder 6, wobei das Anzeigeelement ein Symbol umfasst.

9. Medikamentenabgabevorrichtung für dosierte Tröpfchen nach Anspruch 5, wobei die Öffnung mit einer Vergrößerungslinse versehen ist.

10. Medikamentenabgabevorrichtung für dosierte Tröpfchen nach Anspruch 1, wobei die Vorrichtung ein Inhaliergerät ist.

11. Medikamentenabgabevorrichtung für dosierte Tröpfchen nach Anspruch 1, wobei die Vorrichtung eine Augenspray-Vorrichtung ist.

12. Medikamentenabgabevorrichtung für dosierte Tröpfchen nach Anspruch 1, wobei die Vorrichtung ein Injektor ist.

## Revendications

1. Dispositif d'administration dosée de médicament vaporisé, comprenant :
- un châssis tubulaire globalement allongé (64) possédant des extrémités opposées distale et proximale ;
- un mécanisme d'entraînement agencé à l'intérieur du châssis, comprenant :
- une tige de piston filetée (74) ;
- un écrou d'entraînement (82) relié par pas de vis à la tige de piston filetée ;
- un élément d'entraînement (118) en liaison rotative avec un élément de contrainte à ressort (166) accessible à l'extérieur de l'extrémité distale du châssis (64), ledit élément d'entraînement (118) étant relié à l'écrou d'entraînement (82) par un prolongement d'élément d'entraînement (76), ledit prolongement d'élément d'entraînement (76) et ledit élément d'entraînement (118) étant en interconnexion fonctionnelle pour assurer un blocage unidirectionnel en rotation de l'élément d'entraînement (118) par rapport au prolongement d'élément d'entraînement (76) lorsque ledit élément d'entraînement (118) subit une rotation sous l'effet dudit élément de contrainte à ressort (166), ledit prolongement d'élément d'entraînement (76) et ledit écrou d'entraînement (82) étant en interconnexion fonctionnelle pour assurer un blocage en rotation de l'écrou d'entraînement (82) par rapport au prolongement d'élément d'entraînement (76), l'extrémité distale de l'élément d'entraînement (118) présentant un certain nombre de nervures longitudinales (156), un élément de guidage globalement tubulaire (158) possédant une paroi terminale (160) étant en outre prévu, l'extrémité distale de l'élément d'entraînement (118) présentant les nervures (156) étant en outre conçue pour loger dans un évidement (164) correspondant présent sur l'élément de contrainte à ressort (166) de manière que l'élément de contrainte à ressort (166) est en liaison rotative avec l'élément d'entraînement (118), un ressort (176) étant en outre prévu entre la paroi terminale (160) de l'élément de guidage (158) et une paroi intérieure, dirigée de façon proximale, de l'élément de contrainte à ressort (166), sollicitant ce dernier dans la direction distale ;
- un écrou de guidage (94) présentant des nervures de guidage (96) coopérant avec des rainures longitudinales (98) de la tige de piston (74) pour assurer un blocage en rotation tout en permettant un déplacement longitudinal de la tige de piston par rapport à l'écrou de guidage ;
- l'élément de contrainte à ressort (166) accessible à l'extérieur de l'extrémité distale du châssis (64) et en liaison fonctionnelle avec le mécanisme d'entraînement ;
- un élément à force de ressort (134) possédant une première extrémité reliée à l'élément de contrainte à ressort par l'intermédiaire du mécanisme d'entraînement et une seconde extrémité reliée à un point fixe sur le châssis (64), si bien que ledit élément à force de ressort (134) est contraint lorsque ledit élément de contrainte à ressort (166) est activé ;
- des moyens d'activation manuelle (178, 180, 182, 192) en interconnexion avec faculté de libération vis-à-vis dudit mécanisme d'entraînement et conçus pour interagir avec ledit mécanisme d'entraînement de manière qu'une fois l'élément à force de ressort (134) contraint, toute activation dudit élément de contrainte à ressort (166) soit empêchée ;
ledit dispositif comprenant en outre un mécanisme indicateur (128, 132) en liaison fonctionnelle avec ledit mécanisme d'entraînement et destiné à indiquer visuellement à l'utilisateur du dispositif que ledit élément de contrainte à ressort (166) a été activé pour rendre le dispositif prêt à l'administration d'une dose, et ledit élément d'entraînement (118) présentant une surface (128) dirigée globalement radialement vers l'extérieur et présentant des indications servant à indiquer le statut du dispositif.

2. Dispositif d'administration dosée de médicament vaporisé selon la revendication 1, dans lequel lesdits moyens d'activation manuelle (178, 180, 182, 192) sont en interconnexion avec faculté de libération vis-à-vis dudit prolongement d'élément d'entraînement grâce à des moyens d'emboîtement (198, 200) pour assurer un blocage en rotation du prolongement d'élément d'entraînement (76) dans quelque sens que ce soit lorsque ledit élément d'entraînement (118) subit une rotation et que ledit élément à force de ressort (134) est contraint sous l'effet de l'activation dudit élément de contrainte à ressort (166) pour préparer le dispositif en vue d'une administration d'une dose de médicament.

3. Dispositif d'administration dosée de médicament vaporisé selon la revendication 1, dans lequel la première extrémité dudit élément à force de ressort (134) est reliée à l'élément d'entraînement (118).

4. Dispositif d'administration dosée de médicament vaporisé selon la revendication 1, dans lequel ledit mécanisme indicateur (128, 132) est en liaison fonctionnelle avec ledit élément d'entraînement (118) et conçu pour indiquer visuellement à l'utilisateur du dispositif, à travers le châssis, que ledit élément de contrainte à ressort (166) a été activé pour rendre le dispositif prêt à l'administration d'une dose.

5. Dispositif d'administration dosée de médicament vaporisé selon la revendication 4, dans lequel ledit mécanisme indicateur (128, 132) comprend une surface (128) en liaison fonctionnelle avec ledit élément d'entraînement (118), laquelle surface (128) présente un élément d'indication perceptible, et dans lequel le châssis présente une ouverture.

6. Dispositif d'administration dosée de médicament vaporisé selon la revendication 5, dans lequel ladite surface (128) est agencée fonctionnellement par rapport audit élément d'entraînement (118) de manière que ledit élément d'indication soit visible dans ladite ouverture lorsque ledit élément de contrainte à ressort (166) a été activé pour rendre le dispositif prêt à l'administration d'une dose.

7. Dispositif d'administration dosée de médicament vaporisé selon la revendication 4 ou 6, dans lequel ledit élément d'indication comprend une couleur distincte.

8. Dispositif d'administration dosée de médicament vaporisé selon la revendication 4 ou 6, dans lequel ledit élément d'indication comprend un symbole.

9. Dispositif d'administration dosée de médicament vaporisé selon la revendication 5, dans lequel ladite ouverture présente un verre grossissant.

10. Dispositif d'administration dosée de médicament vaporisé selon la revendication 1, ledit dispositif étant un inhalateur.

11. Dispositif d'administration dosée de médicament vaporisé selon la revendication 1, ledit dispositif étant un dispositif de vaporisation oculaire.

12. Dispositif d'administration dosée de médicament vaporisé selon la revendication 1, ledit dispositif étant un dispositif d'injection.
